# EUROPEAN PATENT APPLICATION

(11) **EP 0 820 748 A1**
(43) Date of publication of application: **28.01.1998**
(21) Application number: 96830403.0
(22) Date of filing: 23.07.1996
(51) Int. Cl.: A61F 13/15

(54) **The use of breathable sanitary napkin for odour control**

(71) Applicant: THE PROCTER & GAMBLE COMPANY, Cincinnati, Ohio 45202 (US)
(72) Inventor: Alemany, Miguel (U.S.), 65015 Montesilvano, (Pescara) (IT)
(74) Representative: Hirsch, Uwe Thomas

(57) **Abstract**

The present invention relates to sanitary napkins or panty liners which are breathable, preferably by the incorporation of a breathable backsheet therein and their use to provide odour control.

## Description

### Field of the Invention

The present invention relates to breathable sanitary napkins and panty liners and their novel use to provide odour control benefits.

### Background of the Invention

Whilst the primary focus of absorbent articles, in particular sanitary napkins remains the ability of these articles to absorb and retain fluids, another important area of development in this field is the control of odourous compounds contained within the absorbed articles during their use. Malodourous compounds typically present in absorbent articles originate from a number of sources. Firstly, the actual components of the fluid discharge such as urine, perspiration, menstrual fluids, menstrual blood and vaginal discharges may themselves contain malodourous compounds. Secondly, malodourous compounds may be generated as a result of the degradation of the components of the fluid discharge. Consequently, there are a wide range of compounds which may be present at some time during the use of an absorbent article which have an associated malodour. These compounds include fatty acids, ammonia, amines, sulphur containing compounds, ketones and aldehydes and numerous derivatives thereof.

The presence and detection of malodourous compounds from absorbent articles during their use, particularly those associated with menstruation may cause the wearer of these products embarrassment. Thus, the prevention of malodour detection from such products is highly desirable.

As a result there are numerous disclosures in the art describing various odour controlling agents for use in absorbent articles in order to address the problem of malodour detection. The odour control agents typically function by physical absorption of the odourous compound or by chemical interaction with the odourous compound or the precursors of odourous compounds, or by masking the odour.

The odour controlling agents are typically classified according to the type of odour the agent is intended to combat. Odours may be classified as being acidic, basic or neutral. Acidic odour controlling agents have a pH greater than 7 and typically include inorganic carbonates, bicarbonates, phosphates and sulphates. Basic odour controlling agents have a pH of less than 7 and include compounds such as citric acid, boric acid and maleic acid. Neutral odour controlling agents have a pH of approximately 7 such as activated carbon, clays, zeolites, silicas and starches. Typically, the preferred agents utilised in absorbent articles are neutral odour controlling agents and examples of such systems are described in EPO 348 978 and EPO 510 619.

However, the above described odour control agents all have associated drawbacks. Many odour control agents do not provide effective odour control over a range of odours. Alternatively, more effective odour control agents are expensive or such as is the case with activated carbon are aesthetically unappealing to the consumer. Furthermore, many of these odour control agents have problems related to their effective incorporation within the absorbent articles. In addition, none of these odour controlling agents combat the malodourous compound formation problem within sanitary napkins by addressing the source of the formation of the odour.

Hence, there still exists a need to provide alternative means to provide odour control in absorbent articles.

It has now been surprisingly found that the use of a breathable sanitary napkin, particularly by the provision of a breathable backsheet provides an odour control benefit. Furthermore, the requirement of additional odour control agents within the sanitary napkin is considerably reduced and preferably the sanitary napkin requires no additional odour controlling agents.

The problem of the intensification of malodours within sanitary napkins from non breathable absorbent articles has been described in EP 171 041. US 4 059 114 discloses the incorporation of antimicrobial agents in sanitary napkins which have vapour permeable backsheets. However, although the presence of these backsheets assist in the prevention of bacteria generation, the document teaches that an odour control agent is an essential component of the sanitary napkin in order to obtain an odour control benefit. Hence, whilst breathable backsheets are known in the prior art, their use as odour control agents has not been previously recognised or disclosed.

It is believed that the odour control performance benefit of a breathable sanitary napkin is due to a number of factors.

Firstly, the breathability of the sanitary napkin facilitates a reduction of the hot, humid and anaerobic environment typically present between the skin of the wearer and the wearer facing surface of the sanitary napkin. This hinders the growth of microorganisms. Microorganisms are responsible for the generation of odourous compounds. Thus the amount of odours associated with the presence of microorganisms is reduced.

Secondly, the reduction in the hot, humid and occlusive environment between the vicinity of the skin of the wearer and the wearer facing surface of the absorbent article itself also reduces the tendency of the wearer of the sanitary napkin to perspire. Consequently, the amount of associated perspiration related odour will be reduced.

Thirdly, due to the breathable nature of the sanitary napkin, the malodourous compounds contained therein may, similar to water vapour and air, be more readily exchanged with the environment. Hence, malodourous compounds and other compounds which form malodourous degradation products present in the article are able to escape therefrom and are dissipated into the surroundings.

Hence the use of a breathable sanitary napkin or panty liner reduces both the detection of malodours compounds and reduces the formation of these compounds themselves in such products.

### Summary of the Invention

The present invention relates to a breathable sanitary napkin or panty liner having a water vapour permeable substrate, preferably by the provision of a breathable backsheet and its use for the reduction and prevention of malodour detection therefrom.

### Detailed Description of the Invention

The present invention relates to sanitary napkins or panty liners having odour control. Typically such products have a wearer facing surface and a garment facing surface and comprise an absorbent material. According to the present invention the sanitary napkin also comprises a water vapour permeable substrate which provides the garment facing surface of the sanitary napkin. The present invention thus relates to the use of said water vapour permeable substrate in a sanitary napkin to provide odour control. The term water vapour permeable substrate as used herein refers to a material which allows the circulation of water vapour and preferably both water vapour and air through said material. The term odour control as used herein refers to a means of reducing, preferably preventing the detection of malodourous compounds from sanitary napkins or panty liners during use.

Typically sanitary napkins and panty liners comprise in addition to said absorbent material, a liquid pervious topsheet and a backsheet, wherein the absorbent material or core is positioned intermediate the topsheet and the backsheet. According to the present invention said water vapour permeable substrate and hence said garment facing surface of the sanitary napkin may thus be provided by the absorbent material. in such an embodiment the sanitary napkin does not comprise a backsheet. The absorbent material or core can be fluffy fibrous absorbent core comprising hydrogel particles if desired, or laminated tissues with or with out particulate materials including hydrogel particles. The absorbent core fibres can be any on those known in the art including cellulose fibres or polymeric fibres rendered absorbent or even non absorbent matrix fibres. Also tissues of sufficient basis weight and absorbency can be used in the absorbent core according to the present invention.

Alternatively, in a more preferred embodiment of the present invention said water vapour permeable substrate and hence the garment facing surface of the absorbent article may be provided by the backsheet. In this embodiment the sanitary napkins or panty liners comprise as a essential component in addition to the absorbent material, a water vapour permeable backsheet, commonly known as a breathable backsheet. The primary role of a backsheet in sanitary napkins is to prevent the extrudes absorbed and contained in the napkin from wetting articles that contact the absorbent product such as underpants, pants, pyjamas and undergarments. in order to achieve this the backsheet typically will extend across the whole of the absorbent structure and may extend into and form part of or all of sideflaps, side wrapping elements or wings. In addition however, breathable backsheets according to the present invention also permit the transfer of water vapour and preferably both water vapour and air through it and thus allow the circulation of air into and out of the backsheet and thus through the sanitary napkin or panty liner itself.

Suitable breathable backsheets for use herein include all breathable backsheets known in the art. In principle there are two types of breathable backsheets, single layer breathable backsheets which are breathable and impervious to liquids and, backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867 US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

According to the present invention the breathable backsheet comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2 dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2 dimensional planar layers of the backsheet may be made of any material known in the art, but are preferably manufactured from commonly available polymeric materials. Suitable materials are for example Gore-tex (TM) or Sympatex (TM) type materials well known in the art for there application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3, 929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core.

Suitable macroscopically expanded films for use herein include films as described in for example in US 637 819 and US 4 591 523.

Suitable monolithic films include Hytrel™ ,available from DuPont Corporation, USA and other such materials as described in Index 93 Congress, Session 7A, "Adding value to Nonwovens", J-C. Cardinal and Y. Trouihet, DuPont Nemours International S.A. Switzerland

According to the present invention the backsheet may comprise in addition to said water vapour permeable layer additional backsheet layers. Said additional layers may be located on either side of said water vapour permeable layer of the backsheet. The additional layers may be of any material, such as fibrous layers or additional water vapour permeable layers as defined herein above. In a particularly preferred embodiment the fibrous layer is a non woven composite comprising at least on layer of meltblown fibres and at least one layer of spun bonded fibres.

In a preferred embodiment of the present invention the breathable backsheet and other components of the sanitary napkin meet the requirements as described in European Patent Application number 96830343.8 such that effective breathability is exhibited throughout the sanitary napkin.

The sanitary napkin or panty liners of the present invention may also comprise in addition to the absorbent core or the absorbent core and breathable backsheet, a topsheet, which may comprise a single layer or a multiplicity of layers. In a preferred embodiment the topsheet comprises a first layer which provides the wearer facing surface of the topsheet and a second layer between the first layer and the absorbent structure/core.

The topsheet provides a layer through which the liquids to be absorbed can penetrate to the absorbent material. The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as non woven fabrics, films or combinations of both. In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314 and US 4 591 523.

The topsheet typically extends across the whole of the absorbent structure and can extend into and form part of or all of the preferred sideflaps, side wrapping elements or wings.

In one embodiment of the present invention the sanitary napkin or panty liner comprises, preferably within the absorbent core additional odour control agents. Any agents known in the art for the control of odour maybe usefully utilised herein. Suitable agents include activated carbon, zeolites, silica, starch, chelating agents, masking agents, ion exchange resins, buffer systems, clay, AGM antimicrobials, cyclodextrin, carboxylic acids, carbonates, bicarbonates, sulphates, phosphates or any mixtures thereof. According to the present invention the amount of such additional odour control agents may be readily determined by the person skilled in the art depending on the end use and the dimensions of the product. However, according to the present invention the amount of said additional odour control agents may be reduced and in a preferred embodiment of the present invention the sanitary napkin is free of additional odour control agents.

According to the present invention the sanitary napkin is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof.

According to the present invention the sanitary napkin may comprise all those features and parts which are typical for these products in the context of their intended use such as wings, side flaps, undergarment adhesive means, release paper, wrapping elements and the like.

## Claims

1. The use of a water vapour permeable substrate in a sanitary napkin or panty liner to provide odour control, said sanitary napkin having a wearer facing surface, and a garment facing surface and comprising an absorbent material, and said water vapour permeable substrate providing said garment facing surface of said sanitary napkin.

2. The use of a breathable sanitary napkin or panty liner to provide odour control.

3. The use according to the either of the claims 1 or 2, wherein said sanitary napkin or panty liner is free of additional odour control agents.

4. The use according to any one of the preceding claims, wherein said sanitary napkin or panty liner comprises a breathable backsheet.

5. The use according to claim 4, wherein said breathable backsheet comprises at least one layer comprising an apertured polymeric film or a 2-dimensional planar apertured film.

6. The use according to claim 5, wherein said layer is a 2-dimensional planar apertured layer, wherein said apertures have an average diameter of from 150 micrometers to 5 micrometers.

7. The use according to claim 5, wherein said layer is an apertured polymeric film, wherein said apertures have an average diameter of from 100 micrometers to 500 micrometers.

8. The use according to claim 5, wherein said breathable backsheet comprises at least two layers, a first layer comprising an apertured layer and a second layer comprising a fibrous layer.

9. The use according to claim 8, wherein said fibrous layer is a non woven composite comprising at least one layer of meltblown fibres and at least one layer of spun bonded fibres.
